# EUROPÄISCHE PATENTSCHRIFT

(11) **EP 1 332 710 B1**
(45) Veröffentlichungstag und Bekanntmachung des Hinweises auf die Patenterteilung: **03.12.2008**
(21) Anmeldenummer: 03002481.4
(22) Anmeldetag: 05.02.2003
(51) Int. Cl.: A61B 1/05, G02B 23/24

(54) **Endoskop mit Seitblickoptik**
Endoscope with side-view optics
Endoscope avec optique pour vue latérale

(30) Priorität: 05.02.2002 DE 10204718
(43) Veröffentlichungstag der Anmeldung: 06.08.2003
(73) Patentinhaber: Zaar, Kersten, 72461 Albstadt (DE)
(72) Erfinder: Zaar, Kersten, 72461 Albstadt (DE)
(74) Vertreter: Schuster, Gregor

(56) Entgegenhaltungen:
- US-A- 2 987 960
- US-A- 4 640 577
- US-A- 5 253 638

## Beschreibung

### Stand der Technik

Die Erfindung geht aus von einem Endoskop mit Seitblickoptik nach der Gattung des Hauptanspruches.

Aus dem Stand der Technik sind Endoskope mit Seitblicksystemen zur Betrachtung und Beurteilung von Rohrinnenwandungen und Rohrfügungen bekannt, mit denen eine gewünschte Umlenkung des Blickfeldes erreichbar ist. Hierzu können optische Systeme verwendet werden, die aus einer an einem Endoskop oder an einer Rohrkamera fest montierbaren Umlenkoptik bestehen, die jedoch keine motorisch angetriebene Drehvorrichtung aufweisen, sodass damit nur ein kleiner Bereich der Rohrinnenwandung untersucht werden kann. Um das Blickfeld der Rohrkamera zu vergrößern, wurde eine Optik mit einer kontinuierlichen 360°-Rundumsicht geschaffen, die neben dem Blick nach vorne einen Blick auf die Rohrinnenwandung erlaubt. Eine derartige Vergrößerung des Blickfeldes bringt aber eine sehr hinderliche Abschwächung der Ausleuchtung der zu untersuchenden Wandbereiche mit sich.

Um zur Verbesserung der Ausleuchtung die vorhandene Lichtmenge auf kleinere Bereiche zu fokussieren und dennoch größere Innenwandbereiche optisch untersuchen zu können, wurden Endoskope vorgeschlagen, bei denen die Kamera auf einer motorisch antreibbaren Schwenk- bzw. Neigeeinrichtung angeordnet ist. Eine derartige Schwenkeinrichtung für ein Endoskop ist aus der DE-OS 43 29 162 bekannt, die aus einem an drei Punkten mit drei piezoelektrischen Antriebselementen verbundenen, scheibenförmigen Endstück besteht, auf dem ein Kamerachip befestigt ist. Je nach Ansteuerung der Antriebselemente kann damit das Endstück um einen Winkel verschwenkt werden, der konstruktionsbedingt sehr viel kleiner als 90° ist.

Aus der US 5,253,638 A ist ein Endoskop mit einem Seitblicksystem bekannt, bei dem das Gehäuse mit dem Spiegel drehbar ist.

In der US 2,987,960 wird ein Endoskop mit mehreren ovalförmigen Spiegeln beschrieben, mit denen unterschiedliche Blickwinkel einstellbar sind.

Dieses Problem, die Blickrichtung um 90° gegenüber der Längsachse des Endoskops verschwenken zu können, wird von einem bekannten Endoskop mit Rohrkamera dadurch gelöst, dass in Aufnahmerichtung vor der Kamera ein motorisch angetriebener und um die Längsachse des Endoskops drehbarer Spiegel angeordnet ist, der gegenüber der Längsachse derart schwenkbar ist, dass er demgegenüber jeden gewünschten Winkel einnehmen kann. Hierbei bildet der Motor mit dem Spiegel eine konstruktive Einheit, die über mehrere Stege mit der Rohrkamera verbunden ist. Insb. bei kleineren Endoskopen dürfen diese Stege eine gewisse Stärke nicht unterschreiten, sodass hierdurch das reflektierte Abbild des zu untersuchenden Objektes gestört werden kann, indem bspw. von einem Steg entscheidende Schadstellen der Rohrinnenwandung verdeckt werden. Außerdem können diese Stege in den Kegel des die Innenwandung beleuchtenden Lichtes gelangen, sodass dadurch Schatten gebildet werden, die ebenfalls dazu führen können, dass Schadstellen des zu untersuchenden Objektes unentdeckt bleiben. Weiterhin besteht die Gefahr, dass die Stege das zur Ausleuchtung dienende Licht reflektieren, sodass eine Videokamera mit automatischer Belichtungseinstellung zwar den stark beleuchteten Steg nicht über den dunkleren Hintergrund, d. h., das zu untersuchende Objekt, aufnimmt.

### Die Erfindung und ihre Vorteile

Das erfindungsgemäße Endoskop mit Seitblickoptik art Anspruch 1 hat demgegenüber den Vorteil, eine endoskopische Betrachtung schwer zugänglicher Objekte zu ermöglichen, bei der das zur Beleuchtung des Objektes dienende Licht ungehindert auf das Objekt gelangt und das zu untersuchende Abbild des Objektes von einer Seitblickoptik ungestört auf das Objekt einer hierbei verwendeten Videokamera reflektiert wird. Das Antriebsmittel weist ein Zahnrad auf, womit der Hohlzylinder über ein auf dessen Zylinderinnenwand angeordnetes Hohlzahnrad in Drehung versetzbar ist. Hierdurch ergibt sich die Möglichkeit, dass entweder nur die Kamera oder zudem auch das Antriebsmittel innerhalb des Hohlzylinders angeordnet werden.

Nach einer vorteilhaften Ausgestaltung der Erfindung ist die Videokamera von einem zylinderförmigen Gehäuse umgeben, das mit dem Hohlzylinder einen Spalt bildet, wobei auf dem Gehäuse mindestens eine Dichtung zur Abdichtung des Spaltes angeordnet ist. Hierdurch wird verhindert, dass in das Innere des Endoskops Feuchtigkeit oder Schmutz eindringt, wodurch dessen Funktionsfähigkeit beeinträchtigt werden kann.

Nach einer weiteren vorteilhaften Ausgestaltung der Erfindung wird als Antriebsmittel ein Elektromotor verwendet. Selbstverständlich sind als Antriebsmittel auch andere technische Lösungen, beispielsweise hydraulische oder pneumatische Antriebe denkbar.

Nach einer weiteren vorteilhaften Ausgestaltung der Erfindung ist zwischen Antriebsmittel und Zahnrad ein Getriebe angeordnet, womit die Drehzahl des Antriebsmittels in geeigneter Weise heruntergesetzt werden kann.

Nach einer weiteren vorteilhaften Ausgestaltung der Erfindung ist die Videokamera mit dem Antriebsmittel mechanisch fest verbunden. Hierdurch wird ermöglicht, den drehbaren Hohlzylinder je nach Ausgestaltung der Erfindung gleitend entweder nur auf dem Gehäuse der Videokamera oder auf dem gemeinsamen Gehäuse von Antriebsmittel und Videokamera zu lagern, was dem erfindungsgemäßen Endoskop eine große mechanische Festigkeit verleiht.

Nach einer weiteren vorteilhaften Ausgestaltung der Erfindung ist die Seitblickoptik mit dem Hohlzylinder über einen halbrohrförmigen Ansatz verbunden, der eine ungehinderte Ausleuchtung und Betrachtung des zu untersuchenden Objektes erlaubt und eine stabile Halterung der Seitblickoptik am Hohlzylinder ergibt.

Nach einer weiteren vorteilhaften Ausgestaltung der Erfindung ist der Spiegel der Seitblickoptik als Prisma ausgebildet, der gegenüber herkömmlichen Spiegeln eine verbesserte Reflektivität aufweist.

Nach einer weiteren vorteilhaften Ausgestaltung der Erfindung kann das Antriebsmittel den Hohlzylinder über ein Reibradgetriebe antreiben, das insb. bei kleinen Endoskopen sehr preisgünstig realisierbar ist.

Nach einer weiteren vorteilhaften Ausgestaltung der Erfindung ist der Elektromotor als Schrittmotor ausgebildet, der aus auf dem Gehäuse angeordneten elektrischen Wicklungen und aus auf dem Hohlzylinder angeordneten und mit den Wicklungen je nach deren Ansteuerung in elektromagnetische Wechselwirkung tretenden Permanentmagneten besteht. Derartige Motoren sind besonders einfach aufgebaut und somit auch preisgünstig herstellbar. Insb. aber erlauben diese Motoreneine sehr genaue Positionierung der erfindungsgemäßen Seitblickoptik.

Nach einer weiteren vorteilhaften Ausgestaltung der Erfindung ist eine Vorrichtung zum Einkoppeln von Licht in den Lichtleiter vorgesehen mit einer derart schwenkbaren Halterung des dem Aufnahmegerät abgewandten Endes des Lichtleiters, dass bei einer ersten Schwenkposition der Halterung eine maximale und bei einer zweiten Schwenkposition der Halterung eine minimale Lichtmenge in die Stirnfläche des in der Halterung befestigten Endes des Lichtleiters einkoppelbar ist. Hierdurch kann die Menge des in den Lichtleiter eingekoppelten und der Beleuchtung des zu untersuchenden Objektes dienenden Lichtes der Struktur dieses Objektes genau angepasst werden.

Nach einer weiteren vorteilhaften Ausgestaltung der Erfindung weist die Vorrichtung eine Lampe mit einem lichtfokussierenden Reflektor auf, ist der Lichtleiter mit in Richtung des Reflektors weisender Stirnfläche in der Halterung befestigt und ist die Halterung um eine zwischen dem Lichtfokus und dem Reflektor liegende, vertikale Achse zwischen der ersten und der zweiten Schwenkposition verschwenkbar. Hierdurch kann auf einfache Weise erreicht werden, dass die Stirnfläche des Lichtleiters je nach Schwenkposition derart in den Lichtfokus des Reflektors gelangt, dass eine genau dosierbare Lichtmenge in den Lichtleiter eingekoppelt wird.

Nach einer weiteren vorteilhaften Ausgestaltung der Erfindung ist zum Verschwenken der Halterung ein motorisch angetriebener Dreharm vorgesehen, der über eine Stange und einen außerhalb der Drehachse der Halterung angeordneten Bolzen mit der Halterung mechanisch verbunden ist. Obwohl der erfindungsgemäße Schwenkmechanismus sehr einfach aufgebaut ist, erlaubt er eine sehr genaue Positionierung der Stirnfläche des Lichtleiters gegenüber dem vom Reflektor abgestrahlten Lichtkegel.

Nach einer weiteren vorteilhaften Ausgestaltung der Erfindung ist das Antriebsmittel über eine Biegewelle und ein Miniaturgetriebe mit dem Hohlzylinder verbunden ist. Dies erlaubt die Anordnung des den Hohlzylinder und damit des die Seitblickoptik in Drehung versetzenden Antriebsmittels im Abstand vom Endoskop an beliebiger Stelle der Sonde und auch außerhalb der Sonde, wodurch die Möglichkeit geschaffen wird, die äußeren Abmessungen des Endoskops weiter zu verkleinern. Die durch die erfindungsgemäße Ausgestaltung bewirkte Trennung der mechanisch festen Verbindung zwischen Antriebsmittel und Videokamera ermöglicht zudem, das Endoskop durch Rohrbögen mit sehr kleinem Radius zu schieben, um deren Innenfläche zu untersuchen.

Nach einer weiteren vorteilhaften Ausgestaltung der Erfindung weist die Kamera ein einstellbares Objektiv auf, das, um eingestellt zu werden, über eine Biegewelle mit einem Elektromotor verbunden ist. Dies ermöglicht, die Einstellung des Objektives der Videokamera von außen zu steuern, wodurch sichergestellt ist, dass die Abbildungen der zu untersuchenden Objekte immer scharf und gut erkennbar sind.

Weitere Vorteile und vorteilhafte Ausgestaltungen der Erfindung sind der nachfolgenden Beispielsbeschreibung, den Zeichnungen und den Ansprüchen entnehmbar.

### Zeichnung

Ein Ausführungsbeispiel der Erfindung ist in den Zeichnungen dargestellt und im Folgenden näher beschrieben.

Es zeigen:
- Fig. 1: ein Endoskop mit einer Seitblickoptik im Schnitt,
- Fig. 2: einen Schnitt durch eine Vorrichtung zum Einkoppeln von Licht in einen Lichtleiter des Endoskops entlang der gebrochenen Linie II-II in Fig. 3,
- Fig. 3: eine Draufsicht der Vorrichtung zum Einkoppeln von Licht in den Lichtleiter und
- Fig. 4: eine Ausgestaltung des Endoskops, bei dem das Objektiv der Videokamera und die Seitblickoptik über Biegewellen angetrieben werden.

### Beschreibung des Ausführungsbeispiels

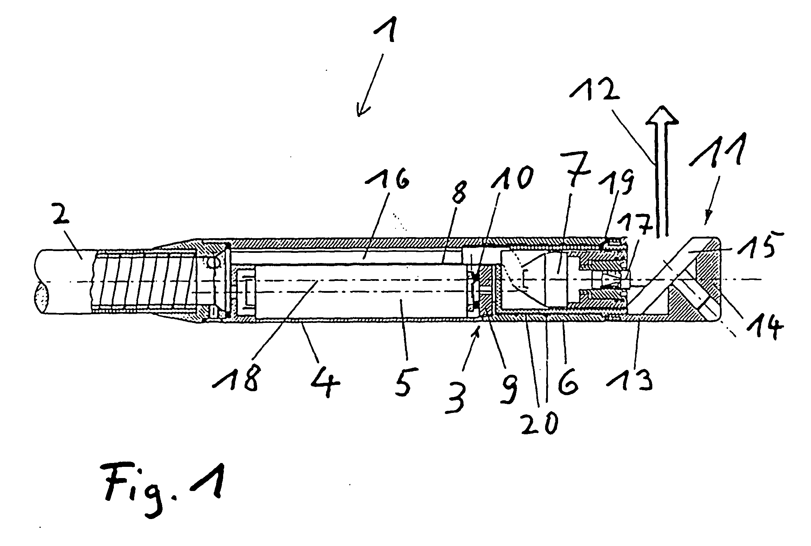

In Figur 1 wird ein Endoskop 1 mit einer Sonde 2 und einem daran befestigten Aufnahmegerät 3 dargestellt. Das Aufnahmegerät 3 weist ein fest mit der Sonde 2 verbundenes Gehäuse 4 für einen Elektromotor 5 und einen drehbar damit verbundenen Hohlzylinder 6 auf, in dessen Inneren eine Videokamera 7 in Miniaturform angeordnet ist, wie sie für Endoskope üblicherweise verwendet und hier nicht näher erläutert wird. Die Videokamera 7 ist über eine in der Sonde 2 geführte und in der Figur nicht dargestellte elektrische Leitung an ein Gerät zur Auswertung der gelieferten Videosignale angeschlossen und mit dem Gehäuse 8 des Elektromotors 5 fest verbunden, der mittels eines Zahnrades 9 somit lediglich den Hohlzylinder 6 in Drehung versetzen kann, der zu diesem Zweck im Bereich des Zahnrades 9 ein nicht näher dargestelltes Hohlzahnrad aufweist. Weiterhin befindet sich zu diesem Zweck zwischen dem Elektromotor 5 und dem Zahnrad 9 ein Getriebe 10, das die Drehzahl des Elektromotors 5 in geeigneter Weise heruntersetzt. Während einer Verdrehung des Hohlzylinders 6 bleibt somit die Videokamera 7 relativ zum Elektromotor 5 unbeweglich in Ruhe. Hierbei kann die Drehbewegung des Elektromotors 5 und damit des Hohlzylinders 6 mittels in der Figur nicht dargestellter in der Sonde 2 geführter Zuleitungen beliebig gesteuert werden. Im vorliegenden Ausführungsbeispiel ist die Videokamera 7 von einem zylinderförmigen Gehäuse 19 umgeben, das mit dem Hohlzylinder 6 einen Spalt bildet. Um zu vermeiden, dass in diesen Spalt Feuchtigkeit oder Schmutz eindringt, wodurch die Funktionsfähigkeit des elektrischen Antriebes (5, 9, 10) beeinträchtigt werden könnte, sind zwischen dem Gehäuse 19 und dem Hohlzylinder 6 Dichtungen 20 angeordnet.

Auf der zur durch den Pfeil 12 angedeuteten Blickrichtung abgewandten Seite ist eine Seitblickoptik 11 an dem Hohlzylinder 6 befestigt, die sich somit beim Verdrehen des Hohlzylinders 6 mitdreht. Die Seitblickoptik 11 besteht im Wesentlichen aus einem halbrohrförmigen Ansatz 13, dessen eine Seite mit dem Hohlzylinder 6 fest verbunden ist und an dessemn anderen Ende sich ein Haltegestell 14 für einen Spiegel 15 befindet, der im vorliegenden Ausführungsbeispiel als Prisma ausgebildet ist. Mit der Längsachse 18 des Endoskops 1 schließt im vorliegenden Ausführungsbeispiel der Spiegel 15 einen Winkel von 45° ein. Je nach Anwendungszweck des Endoskops 1 kann dieser Winkel aber auch einen anderen Wert aufweisen. Wird Licht über einen seitlich am Elektromotor 5 vorbeigeleiteten Lichtleiter 16 der Videokamera 7 zugeführt und über die Stirnfläche des Lichtleiters 16 in nicht näher dargestellter Weise auf den Spiegel 15 gestrahlt, wird hierdurch das Licht umgelenkt und in Richtung des Pfeils 12 auf das näher zu untersuchende Objekt projiziert. Das Abbild des hierdurch beleuchteten Objektes wird vom Spiegel 15 auf das Objektiv 17 der Videokamera 7 reflektiert und kann von dieser somit aufgenommen werden. Insb. wenn auf diese Weise die Innenwand eines Rohres optisch abgetastet wird, kann durch ein vom Elektromotor 5 über den Hohlzylinder 6 bewirktes Verdrehen der Seitblickoptik 11 die gesamte Innenwand des zu begutachtenden Rohres untersucht werden.

Der Elektromotor 5 kann nicht nur, wie in der Figur dargestellt, hinter der Videokamera 7 sondern auch daneben angeordnet sein. In diesem zeichnerisch nicht dargestellten Fall ist die Videokamera 7 in dem gleichen Gehäuse angeordnet, wie der Elektromotor 5, und sind Videokamera 7 und Elektromotor 5 innerhalb des per Zahn- oder Reibrad vom Elektromotor 5 antreibbaren Hohlzylinders 6 angeordnet, an dem die Seitblickoptik 11 befestigt ist.

Denkbar ist hierbei auch, den Elektromotor 5 durch einen Schrittmotor zu ersetzen, bei dem auf dem Gehäuse 19 der Videokamera 7 elektrische Wicklungen angebracht sind und der drehbare Hohlzylinder 6 mit Permanentmagneten versehen ist. Je nach gewünschter Blickrichtung 12 werden die entsprechenden elektrischen Wicklungen angesteuert, die auf die Dauermagneten dann anziehende oder abstoßende Kräfte ausüben und damit die Seitblickoptik 11 in die gewünschte Position drehen.

Das Endoskop 1 mit der erfindungsgemäßen Seitblickoptik 11 hat hierbei insb. den Vorteil, dass durch deren Befestigung auf der der Blickrichtung 12 abgewandten Seite mittels des halbrohrförmigen Ansatzes 13 keine störenden Befestigungsstege den Strahlengang und die Abbildung des zu untersuchenden Objektes stören.

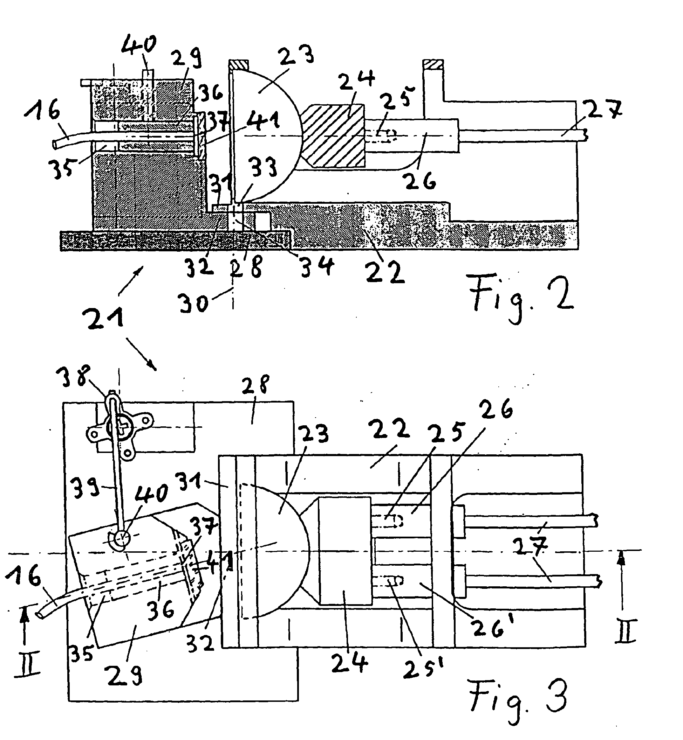

In den Figuren 2 und 3 ist eine Vorrichtung 21 dargestellt, mit deren Hilfe das Licht in den Lichtleiter 16 einkoppelbar ist, das der Beleuchtung des mit dem Endoskop 1 zu untersuchenden Objektes dient. In Fig. 3 ist die Vorrichtung 21 in Draufsicht dargestellt, und Fig. 2 zeigt einen Schnitt durch die Vorrichtung 21 entlang der in Fig. 3 eingezeichneten gebrochenen Linie II-II. Die Vorrichtung 21 besteht aus einer in einem Gestellt 22 befestigten Lampe, von der der Einfachheit halber in den Fig. 2 und 3 lediglich der Reflektor 23 eingezeichnet ist. Dieser ist befestigt an einer Lampenhalterung 24, die als elektrischer Stecker ausgebildet ist, dessen Anschlussstifte 25 und 25' in je einer Steckerbuchse 26, 26' mit Anschluss an das elektrische Netz 27 stecken.

Auf der dem Netzanschluss 27 abgewandten Seite ist an dem Gestellt 22 eine Platte 28 befestigt, auf der eine Halterung 29 für das Ende des Lichtleiters 16 um eine vertikale Achse 30 schwenkbar gelagert ist. Ein Vorsprung 31 des Gestells 22 bildet hierbei mit dem rechten Ende der Platte 28 eine Ausnehmung, in der ein Vorsprung 32 der Halterung 29 eingeführt und mittels eines in den Figuren nicht dargestellten, in die Bohrung 33 des Gestells 22 und die Bohrung 34 des Vorsprunges 32 eingeführten Bolzens mit einer parallel zur Achse 30 liegenden Längsachse derart gehalten wird, dass Schwenkbewegungen der Halterung 29 um die Achse 30 möglich sind.

Zur Fixierung des Endes des Lichtleiters 16 ist in der Halterung 29 eine Bohrung 35 mit einem größeren Durchmesser eingearbeitet, in den ein zylinderförmiges Haltestück 36 passt, in dem das Ende des Lichtleiters 16 derart angeordnet ist, dass dessen Stirnfläche 37 mit der Vorderseite des Haltestückes 36 plan abschließt. Gegenüber der Lame 23 ist die Stirnfläche 37 des Lichtleiters 16 derart angeordnet, dass je nach Schwenkstellung der Halterung 29 mehr oder weniger Licht der Lampe 23 in den Lichtleiter 16 einkoppelbar ist. Zum Schutz der Stirnfläche 37 ist davor eine Glasplatte 41 angeordnet.

Bewirkt wird die Schwenkbewegung der Halterung 29 von einem Antrieb, der einen Dreharm 38 aufweist, der über eine Stange 39 und einen Bolzen 40 mit der Halterung 29 mechanisch verbunden ist.

Bei der in Fig. 3 dargestellten Schwenk-Stellung der Halterung 29, weist die Stirnfläche 37 des Lichtleiters 16 gegenüber der Lampe 23 eine Position auf, bei der hiervon eine maximale Lichtmenge in den Lichtleiter 16 einkoppelbar ist. Bei einer Drehung des Dreharmes 38 gegen den Uhrzeigersinn wird hiervon auch die Halterung 29 gegen den Uhrzeigersinn verschwenkt. Dies bewirkt, dass die Stirnfläche 37 des Lichtleiters 16 aus dem vom Reflektor 23 gebildeten Lichtfokus herauswandert, und damit die Menge des in den Lichtleiter 16 eingekoppelten Lichtes abnimmt. Es kann also über eine Verstellung des Dreharmes 38 die Menge des in den Lichtleiter 16 eingekoppelten Lichtes exakt eingestellt und der Art des vom Endoskop 1 zu untersuchenden Objektes genau angepasst werden.

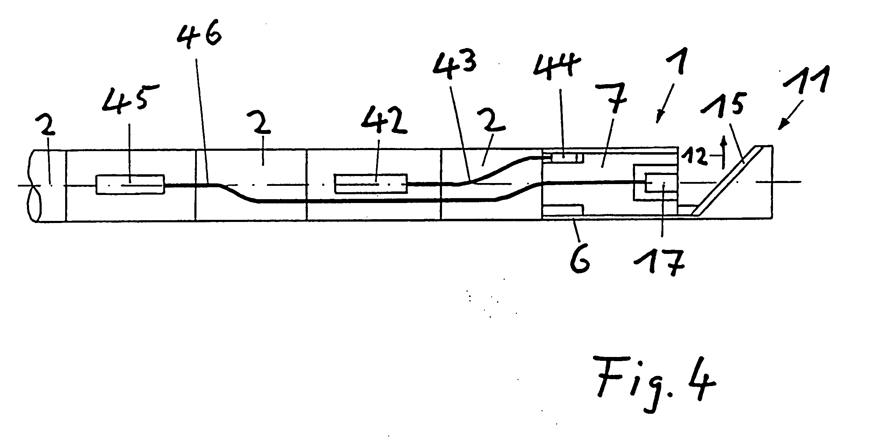

In Figur 4 ist eine weitere Ausgestaltung des erfindungsgemäßen Endoskops mit Seitblickoptik 11 dargestellt. Wie beim Ausführungsbeispiel gemäß Figur 1 ist auch hierbei die Videokamera 7 mit ihrem Objektiv 17 unbeweglich mit der Sonde 2 verbunden und innerhalb des drehbar gelagerten Hohlzylinders 6 angeordnet, der auf der der Blickrichtung 12 abgewandten Seite mit der den Spiegel 15 tragenden Seitblickoptik 11 verbunden ist.

Im Ausführungsbeispiel gemäß Figur 4 aber wird der Hohlzylinder 6 von einem im Abstand zur Videokamera 7 in der Sonde 2 angeordneten Elektromotor 42 in Drehung versetzt, wobei der Elektromotor 42 über eine Biegewelle 43 ein Miniaturgetriebe 44 antreibt, womit dann die Rotation des Hohlzylinders 6 bewirkt wird. Hierbei kann der Elektromotor 42 auch außerhalb der Sonde 2 nahe dem Sondeneingang angeordnet sein.

Auf dieselbe Weise wird zum Zweck der Scharfeinstellung das Objektiv 17 über eine Biegewelle 46 von einem Elektromotor 45 angetrieben. Die Energieversorgung und die Steuerung der Elektromotoren 42 und 45 geschieht in bekannter Weise unter Verwendung elektrischer Leitungen und ist in Figur 4 nicht dargestellt. Ebenso ist auf eine Detaildarstellung der aus dem Stand der Technik bekannten Biegewelle 43 und 46 verzichtet worden, die ähnlich einer Tachowelle aus einem Plastikschlauch mit einer darin drehbar gelagerten Antriebsspirale bestehen kann. Letztlich sei darauf verwiesen, dass die Länge der zwischen der Videokamera 7 und dem Elektromotor 42 und der zwischen den Elektromotoren 42 und 45 befindlichen Sondenabschnitte 2 variabel ist dem entsprechenden Verwendungszweck des Endoskops angepasst werden kann. Hierbei kann der Elektromotor 45 auch außerhalb der Sonde 2 nahe dem Sondeneingang angeordnet sein. Außerdem kann es für bestimmte Verwendungszwecke vorteilhaft sein, die Elektromotore 5, 42 und/oder 45 durch Motoren, die durch unter Druck gebrachte Medien angetrieben werden (z. B. Druckluftmotoren) zu ersetzen.

### Bezugszahlenliste

- 1: Endoskop
- 2: Sonde
- 3: Aufnahmegerät
- 4: Gehäuse
- 5: Elektromotor
- 6: Hohlzylinder
- 7: Videokamera
- 8: Gehäuse des Elektromotors
- 9: Zahnrad
- 10: Getriebe
- 11: Seitblickoptik
- 12: Pfeil, Blickrichtung
- 13: Ansatz
- 14: Haltegestell
- 15: Spiegel
- 16: Lichtleiter
- 17: Objektiv
- 18: Längsachse
- 19: Gehäuse der Videokamera
- 20: Dichtung
- 21: Vorrichtung zum Einkoppeln von Licht in 16
- 22: Gestell
- 23: Lampe, Reflektor
- 24: Lampenhalterung
- 25, 25': Anschlussstift
- 26, 26': Steckerbuchse

- 27: elektrisches Netz
- 28: Platte
- 29: Halterung
- 30: Achse
- 31: Vorsprung von 22
- 32: Vorsprung von 29
- 33: Bohrung
- 34: Bohrung
- 35: Bohrung
- 36: Haltestück
- 37: Stirnfläche von 16
- 38: Dreharm
- 39: Stange
- 40: Bolzen
- 41: Glasplatte
- 42: Elektromotor für die Seitblickoptik 11
- 43: Biegewelle für die Seitblickoptik 11
- 44: Miniaturgetriebe zum Antrieb der Seitblickoptik 11
- 45: Elektromotor zum Einstellen des Objektives 17
- 46: Biegewelle für das Objektiv 17

## Patentansprüche

1. Endoskop (1) mit Seitblickoptik (11),
- die von einem Antriebsmittel (5, 42) in Drehung versetzbar ist, und
- die einen Spiegel (15) zum Umlenken und Projizieren von Licht in einer vorgegebenen Blickrichtung (12) auf ein zu untersuchendes Objekt und zum Reflektieren des Abbildes des Objektes auf ein Objektiv (17) einer im Endoskop (1) angeordneten Videokamera (7) aufweist,
wobei
- sich die Videokamera (7) im Inneren eines drehbar gelagerten und vom Antriebsmittel (5, 42) in Drehung versetzbaren Hohlzylinder (6) befindet,
- die Seitblickoptik (11) auf der der Blickrichtung (12) abgewandten Seite mit dem Hohlzylinder (6) mechanisch verbunden ist,
**dadurch gekennzeichnet,**
- **dass** das Antriebsmittel (5, 42) in Aufnahmerichtung der Videokamera (7) hinter oder neben der Videokamera (7) angeordnet ist und
- **dass** das Antriebsmittel (5) ein Zahnrad (9) aufweist, womit der Hohlzylinder (6) über ein auf dessen Zylinderinnenwand angeordnetes Hohlzahnrad in Drehung versetzbar ist.

2. Endoskop nach Anspruch 1,
**dadurch gekennzeichnet,**
**dass** die Videokamera (7) von einem zylinderförmigen Gehäuse (19) umgeben ist, das mit dem Hohlzylinder (6) einen Spalt bildet, und dass auf dem Gehäuse (19) mindestens eine Dichtung (20) zur Abdichtung des Spaltes angeordnet ist.

3. Endoskop nach Anspruch 1,
**dadurch gekennzeichnet,**
**dass** das Antriebsmittel ein Elektromotor (5, 42) ist.

4. Endoskop nach Anspruch 1,
**dadurch gekennzeichnet,**
**dass** zwischen Antriebsmittel (5) und Zahnrad (9) ein Getriebe (10) angeordnet ist.

5. Endoskop nach einem der vorhergehenden Ansprüche,
**dadurch gekennzeichnet,**
**dass** die Videokamera (7) mit dem Antriebsmittel (5) mechanisch fest verbunden ist.

6. Endoskop nach einem der vorhergehenden Ansprüche,
**dadurch gekennzeichnet,**
**dass** die Seitblickoptik (11) mit dem Hohlzylinder (6) über einen halbrohrförmigen Ansatz (13) verbunden ist.

7. Endoskop nach einem der vorhergehenden Ansprüche,
**dadurch gekennzeichnet,**
**dass** der Spiegel (15) der Seitblickoptik (11)als Prisma ausgebildet ist.

8. Endoskop nach Anspruch 1 oder 2,
**dadurch gekennzeichnet,**
**dass** das Antriebsmittel (5) den Hohlzylinder (6) über ein Reibradgetriebe antreibt.

9. Endoskop nach Anspruch 3,
**dadurch gekennzeichnet,**
**dass** der Elektromotor (5) als Schrittmotor ausgebildet ist, der aus auf dem Gehäuse (19) angeordneten elektrischen Wicklungen und aus auf dem Hohlzylinder (6) angeordneten und mit den Wicklungen je nach deren Ansteuerung in elektromagnetische Wechselwirkung tretenden Permanentmagneten besteht.

10. Endoskop nach einem der vorhergehenden Ansprüche,
**gekennzeichnet durch** eine Vorrichtung (21) zum Einkoppeln von Licht in den Lichtleiter (16) mit einer derart schwenkbaren Halterung (29) des dem Aufnahmegerät (3) abgewandten Endes des Lichtleiters (16), dass bei einer ersten Schwenkposition der Halterung (2) eine maximale und bei einer zweiten Schwenkposition der Halterung (29) eine minimale Lichtmenge in die Stirnfläche (37) des in der Halterung (29) befestigten Endes des Lichtleiters (16) einkoppelbar ist.

11. Endoskop nach Anspruch **10**,
**dadurch gekennzeichnet,**
**dass** die Vorrichtung (21) eine Lampe mit einem lichtfokussierenden Reflektor (23) aufweist, dass der Lichtleiter (16) mit in Richtung des Reflektors (23) weisender Stirnfläche (37) in der Halterung (29) befestigt ist, und dass die Halterung (29) um eine zwischen dem Lichtfokus und dem Reflektor (23) liegende, vertikale Achse (30) zwischen der ersten und der zweiten Schwenkposition verschwenkbar ist.

12. Endoskop nach Anspruch **10** oder **11**,
**dadurch gekennzeichnet,**
**dass** zum Verschwenken der Halterung (29) ein motorisch angetriebener Dreharm (38) vorgesehen ist, der über eine Stange (39) und einen außerhalb der Achse (30) angeordneten Bolzen (40) mit der Halterung (29) mechanisch verbunden ist.

13. Endoskop nach Anspruch 1,
**dadurch gekennzeichnet,**
**dass** das Antriebsmittel (42) über eine Biegewelle (43) und ein Miniaturgetriebe (44) mit dem Hohlzylinder (6) verbunden ist.

14. Endoskop nach Anspruch 1 oder **13**,
**dadurch gekennzeichnet,**
**dass** die Kamera (7) ein einstellbares Objektiv (17) aufweist, das, um eingestellt zu werden, über eine Biegewelle (46) mit einem Elektromotor (45) verbunden ist.

## Claims

1. An endoscope (1) with sideview optics (11),
- which is able to be set in rotation by a drive means (5, 42) and
- which has a mirror (15) for deflection and projection of light in a predetermined direction of view (12) onto an object to be examined and for reflecting of the image of the object to a lens (17) of a video camera (7) arranged in the endoscope (1),
wherein
- the video camera (7) is located in the interior of a rotatably mounted hollow cylinder (6) that can be set in rotation by the drive means (5, 42),
- the sideview optics (11) is mechanically connected with the hollow cylinder (6) on the side facing away from the direction of view (12),
**characterized in that**
- the drive means (5, 42) is arranged behind or next to the video camera (7) in the photographing direction of the video camera (7) and
- the drive means (5) has a gear (9) by which the hollow cylinder (6) is able to be set in rotation via a hollow gear arranged on its cylinder inner wall.

2. The endoscope according to Claim 1,
**characterized in that**
the video camera (7) is surrounded by a cylinder-shaped casing (19), which forms a clearance with the hollow cylinder(6) and that on the casing (19) at least one gasket (20) is arranged for sealing of the clearance.

3. The endoscope according to Claim 1,
**characterized in that**
the drive means is an electric motor (5, 42).

4. The endoscope according to Claim 1,
**characterized in that**
a transmission (10) is arranged between the drive means (5) and the gear (9).

5. The endoscope according to any of the preceding claims, **characterized in that**
the video camera (7) is securely connected mechanically with the drive means (5).

6. The endoscope according to any of the preceding claims,
**characterized in that**
the sideview optics (11) is connected with the hollow cylinder (6) by means of a semi-tubular attachment (13) .

7. The endoscope according to any of the preceding claims,
**characterized in that**
the mirror (15) of the sideview optics (11) is constructed as a prism.

8. The endoscope according to Claim 1 or 2,
**characterized in that**
the drive means (5) drives the hollow cylinder (6) by means of a friction gear.

9. The endoscope according to Claim 3,
**characterized in that**
the electric motor (5) is constructed as a stepping motor, which consists of electrical windings arranged on the casing (19) and of permanent magnets arranged on the hollow cylinder (6) that enter into electromagnetic interaction with the windings depending on their drive.

10. The endoscope according to any of the preceding claims,
**characterized by**
a device (21) for the coupling of light to the fibre optic light guide (16) with a mounting support (29) of the end of the fibre optic light guide (16) facing away from the photo camera (3) that is swivellable in such a way that in a first swivel position of the mounting support (29) a maximum and in a second swivel position of the mounting support (29) a minimum light quantity can be coupled in the frontal area (37) of the end of the fibre optic light guide (16) attached in the mounting support (29).

11. The endoscope according to Claim 10,
**characterized in that**
the device (21) has a lamp with a light-focusing reflector (23), that the fibre optic light guide (16) is attached in the mounting support (29) with the frontal area (37) pointing in the direction of the reflector (23), and that the mounting support (29) can be swivelled around a vertical axis (30) lying between the light focus and the reflector (23) between the first and the second swivel positions.

12. The endoscope according to Claim 10 or 11,
**characterized in that**
a motor-driven rotating arm (38) is provided for swivelling the mounting support (29), which is mechanically connected with the mounting support (29) by means of a rod (39) and a bolt (40) arranged outside the axis (30).

13. The endoscope according to Claim 1,
**characterized in that**
the drive means (42) is connected with the hollow cylinder (6) by means of a flexible shaft (43) and a miniature gear (44).

14. The endoscope according to Claim 1 or 13,
**characterized in that**
the camera (7) has an adjustable lens (17) which, in order to be adjusted, is connected by means of a flexible shaft (46) to an electric motor (45).

## Revendications

1. Endoscope (1) avec optique pour vue latérale (11),
- pouvant être mise en rotation par des moyens d'entraînement (5, 42), et
- comportant un miroir (15) pour dévier et projeter la lumière dans un axe de vision (12) prédéfini sur un objet à examiner, et pour réfléchir l'image de l'objet sur un objectif (17) d' une caméra vidéo (7) disposée dans l'endoscope (1),
- la caméra vidéo (7) étant située à l'intérieur d'un cylindre creux (6) monté de façon rotative et pouvant être mis en rotation par le moyen d'entraînement (5, 42),
- l'optique pour vue latérale (11) étant mécaniquement reliée au le cylindre creux (6) du côté détourné de l'axe de vision (12),
**caractérisé en ce que**
- le moyen d'entraînement (5, 42) est disposé dans l'axe de prise de vue de la caméra vidéo (7), derrière ou à côté de la caméra vidéo (7), et
- le moyen d'entraînement (5) comporte une couronne dentée (9) permettant de mettre en rotation le cylindre creux (6), par une couronne dentée disposée sur la paroi intérieure de cylindre de celui-ci.

2. Endoscope selon la revendication 1,
**caractérisé en ce que**
la caméra vidéo (7) est entourée par un boîtier (19) cylindrique formant un espace par rapport au cylindre creux (6), et **en ce qu'**au moins un moyen d'étanchéité (20) est prévu sur le boîtier (19) pour l'étanchement de l'espace.

3. Endoscope selon la revendication 1,
**caractérisé en ce que**
le moyen d'entraînement est un moteur électrique (5, 42).

4. Endoscope selon la revendication 1,
**caractérisé en ce que**
un engrenage (10) est disposé entre le moyen d'entraînement (5) et la roue dentée (9).

5. Endoscope selon l'une des revendications précédentes,
**caractérisé en ce que**
la caméra vidéo (7) est fixement et mécaniquement reliée au moyen d'entraînement (5).

6. Endoscope selon l'une des revendications précédentes,
**caractérisé en ce que**
l'optique pour vue latérale (11) est reliée au cylindre creux (6) par le biais d'un embout semi-tubulaire (13).

7. Endoscope selon l'une des revendications précédentes,
**caractérisé en ce que**
le miroir (15) de l'optique pour vue latérale (11) est conçu comme un prisme.

8. Endoscope selon la revendication 1 ou 2,
**caractérisé en ce que**
le moyen d'entraînement (5) entraîne le cylindre creux (6) par le biais d'une roue de friction.

9. Endoscope selon la revendication 3,
**caractérisé en ce que**
le moteur électrique (5) est conçu comme un moteur pas à pas, constitué d'enroulements électriques disposés sur le boîtier (19) et d'aimants permanents disposés sur le cylindre creux (6) et entrant en interaction électromagnétique avec les enroulements, en fonction de la commande de ceux-ci.

10. Endoscope selon l'une des revendications précédentes,
**caractérisé par** un dispositif (21) permettant de coupler la lumière dans le conducteur de lumière (16), avec un support (29) pour l'extrémité du conducteur de lumière (16) détournée de l'appareil de prise de vue (3), qui peut être pivoté de telle manière que dans une première position de pivotement du support (2), une quantité maximale de lumière peut être couplée dans la surface frontale (37) de l'extrémité du conducteur de lumière (16) qui est fixée dans le support (29), et une quantité minimale de lumière dans une deuxième position de pivotement du support (29).

11. Endoscope selon la revendication 10,
**caractérisé en ce que**
le dispositif (21) comporte une lampe avec un réflecteur (23) focalisant la lumière, **en ce que** le conducteur de lumière (16) est fixé dans le support (29), avec sa surface frontale (37) tournée vers le réflecteur (23), et **en ce que** le support (29) peut pivoter entre la première et le deuxième position de pivotement, autour d'un axe vertical (30) s'étendant entre le point focal de la lumière et le réflecteur (23).

12. Endoscope selon la revendication 10 ou 11,
**caractérisé en ce que**
pour le pivotement du support (29), il est prévu un bras rotatif (38) entraîné de façon motorisée, mécaniquement relié au support (29) par le biais d'une tige (39) et d'un boulon (40) disposé à l'extérieur de l'axe (30).

13. Endoscope selon la revendication 1,
**caractérisé en ce que**
le moyen d'entraînement (42) est relié au cylindre creux (6) par le biais d'un arbre flexible (43) et d'un engrenage miniature (44).

14. Endoscope selon la revendication 1 ou 13,
**caractérisé en ce que**
la caméra vidéo (7) comporte un objectif ajustable (17), qui, pour pouvoir être ajusté, est relié à un moteur électrique (45) par le biais d'un arbre flexible (46).
